# EUROPEAN PATENT SPECIFICATION

(11) **EP 1 511 462 B1**
(45) Date of publication and mention of the grant of the patent: **18.07.2007**
(21) Application number: 03732474.6
(22) Date of filing: 27.05.2003
(51) Int. Cl.: A61K 31/045, A61P 17/00, A61P 17/16

(54) **USE OF DOLICHOL FOR THE PREVENTION OF ACUTE AND CHRONIC SKIN DAMAGE CAUSED BY EXPOSURE TO SUNLIGHT**
VERWENDUNG VON DOLICHOL ZUR VORBEUGUNG AKUTER UND CHRONISCHER HAUTSCHÄDEN VERURSACHT DURCH SONNENLICHT
UTILISATION DU DOLICHOL POUR LA PRÉVENTION DES LÉSIONS CUTANÉES AIGUES ET CHRONIQUES DUES À L'EXPOSITION AU SOLEIL

(30) Priority: 04.06.2002 IT MI20021204
(43) Date of publication of application: 09.03.2005
(73) Proprietor: ABIOGEN PHARMA S.p.A., 56014 Ospedaletto (Pisa) (IT)
(72) Inventor: BERGAMINI, Ettore, I-56123 Pisa (IT); GORI, Zina, I-56035 Lari (IT); LENCI, Francesco, I-56127 Pisa (IT); SGARBOSSA, Antonella, I-56124 Pisa (IT); CHIELLINI, Emo, I-56127 Pisa (IT); BIZZARRI, Ranieri, I-56021 Cascina (IT)
(74) Representative: Minoja, Fabrizio
(86) International application number: PCT/EP2003/005550
(87) International publication number: WO 2003/101413

(56) References cited:
- EP-A- 0 095 133
- EP-A- 0 149 847
- US-A- 4 812 443
- US-A- 5 575 994
- US-B1- 6 261 575
- PATENT ABSTRACTS OF JAPAN vol. 011, no. 163 (C-424), 26 May 1987 (1987-05-26) & JP 61 293905 A (KANEBO LTD), 24 December 1986 (1986-12-24)
- PATENT ABSTRACTS OF JAPAN vol. 011, no. 379 (C-463), 10 December 1987 (1987-12-10) & JP 62 148415 A (KANEBO LTD), 2 July 1987 (1987-07-02)
- DINI B ET AL: "Effects of ageing and increased haemolysis on the levels of dolichol in rat spleen." EXPERIMENTAL GERONTOLOGY, vol. 37, no. 1, December 2001 (2001-12), pages 99-105, XP001154275 ISSN: 0531-5565

## Description

The present invention relates to dermatological compositions containing dolichol as an active agent, in a particular to compositions which prevent the harmful action of sun light, and are also useful to regularise skin pigmentation and sebum secretion. This activity is performed by enhancing the skin natural defences.

### INTRODUCTION

The skin consists of a set of cells whose state is reflected by the external appearance of the epidermis. It is continually exposed to the harmful effects of reactive substances (free radicals), which may be endogenous (products of skin cell metabolism) or originate from the outside world (high oxygen concentrations; radical generators present in the environment, including smoke, sunlight, etc.) and are responsible for harmful acute and chronic symptoms, and in particular for intrinsic and extrinsic skin aging, respectively. The skin aging process is caused by a reduction in the functions of the dermis and the epidermis, especially nutrition, respiration, moisturisation, regeneration and protection of the skin tissues. Skin aging is a natural phenomenon; its causes are variable, and the speed with which it takes place varies from person to person, depending partly on the defences of the skin and the function of the adnexal glands, especially the sebaceous glands, which deposit protective materials on the skin surface. A slowing in the functions of some cells which play a very important part in maintaining the integrity of the epidermal structure generally begins to appear at around the age of 30, but is due to a number of factors, such as genetic background, general state of health, lifestyle and environment (A. Chuttani and B.A. Gilchrest in. E. Masoro (ed). Handbook of Physiology Section 11: Aging. 1995, pp. 309-324).

Particular concern has been aroused by the change in composition of the sunlight spectrum that reaches the earth surface due to the thinning of the ozone layer. This has led to an increase in the proportion of ultraviolet radiation reaching the earth surface, namely UVA (320-400 nm), UVB (290-320 nm) and UVC (200-290 nm), which once constituted 3-5% of solar radiation energy but are now increasing so much that there has been a significant rise in the frequency of malignant skin tumours. Carcinogenic action is mainly attributable to UVB rays, which act as complete carcinogens, ie. have both an initiating and a promoting action. The tumours caused by UV rays include basal cell and squamous cell carcinomas, and probably also the far more serious melanomas. Excessive exposure to sunlight in the first few years of life is believed to constitute a particular risk for the appearance of melanoma.

Photoprotection measures, including application to exposed surfaces of sunscreens, namely substances able to absorb or block UVA and UVB radiation to varying extents, are increasingly recommended to prevent the damage caused by over-exposure to UV rays (reduction in the skin immune defences, alterations in skin pigmentation, premature aging and alterations in dermal capillaries). There are two main types of sunscreen: chemical and physical. Chemical sunscreens are chromophores able to absorb energy in the UVB and UVA regions, thus reducing the number of photons that penetrate the skin. The main categories of chemical sunscreens currently used are para-aminobenzoic acid and its esters; benzophenones, anthranylates, cinnamates and salicylates. Physical sunscreens are mixtures opaque to light, based on zinc oxide, talc and titanium oxide, with good reflecting power. In addition to the light absorption or reflection characteristics of the product, the efficacy of sunscreens depends on a number of factors, including the amount of product intentionally applied (the most important factor) and the distribution procedures (Diffey BL, Sunscreens and UVA protection: a major issue of minor importance. Photochem Photobiol 2001 Jul; 74(1):61-3).

All currently known sunscreens present drawbacks which can make their use harmful, especially over the long term. One drawback is diacutaneous absorption of the constituents of the preparation, with harmful consequences in the skin or at systemic level.

Recent studies have given rise to new concern about the consequences of the increasing dissemination in the environment of many synthetic UV filters which are not easily biodegradable. Among them, some widely used substances (benzophenone-3 (Bp-3), homosalate (HMS), 4-methylbenzylidene camphor (4-MBC), octyl-methoxycinnamate (OMC) and octyl-dimethyl-PABA (OD-PABA) have oestrogenic effects, which can be dangerous because of their bioaccumulation capacity in a wide variety of living species (Schlumpf M, Cotton B, Conscience M, Haller V, Steinmann B, Lichtensteiger W. In vitro and in vivo estrogenicity of UV screens. Environ Health Perspect 2001 Mar; 109(3):239-44).

All these drawbacks can be overcome by using natural antioxidants. If administered orally, they reach the skin through the bloodstream, and can also reach the surface of the epidermis if they are excreted with the sebum. Sebum continually deposits antioxidant substances such as Vitamin E on the skin surface. Applications of Vitamin E to the skin surface have a significant photoprotective effect, but it is short-lived, because alpha-tocopherol is rapidly consumed by photo-oxidation and polymerisation (Krol ES, Kramer-Stickland KA, Liebler DC. Photoprotective actions of topically applied vitamin E. Drug Metab Rev 2000 Aug-Nov; 32(3-4):413-20). Other natural antioxidant substances currently being studied are carotenes and polyunsaturated fatty acids (Glaser DA, Rogers C. Topical and systemic therapies for the aging face. Facial Plast Surg Clin North Am 2001 May; 9(2):189-96). Drinking infusions of green tea, and above all local application of extracts containing the polyphenol components (-)-epigallocatechin-3-gallate (EGCG) and (-)-epicatechin-3-gallate (ECG) have also proved effective (Ahmad N, Mukhtar H., Cutaneous photochemoprotection by green tea: a brief review. Skin Pharmacol Appl Skin Physiol 2001 Mar-Apr; 14(2):69-76; Elmets CA, Singh D, Tubesing K, Matsui M, Katiyar S, Mukhtar H. Cutaneous photoprotection from ultraviolet injury by green tea polyphenols. J Am Acad Dermatol 2001 Mar; 44(3):425-32).

US 6,261,575 discloses dermatological preparations which contain sterols and their biochemical precursors in combination with ubiquinones and/or plastoquinones. Various substances, such as substances with an aerobic cellular energy metabolism, can be added to these formulations; such substances surprisingly include dolichol, a product of the mevalonate metabolic pathway, high concentrations of which are present in human sebum (0.2 mg/g of fresh weight).

JP 61293905 discloses cosmetic compositions for the prevention of skin ageing which contain dolichol; however, this document is silent on the photoprotective effect of this active ingredient.

### DESCRIPTION OF THE INVENTION

It has now been found that dolichol as it possesses antioxidant properties and can be used as a sunscreen to prevent acute or chronic skin damage caused by exposure to sunlight.

The present invention relates to dermatological compositions containing dolichol for the prevention of acute and chronic skin damage caused by exposure to sunlight. Dolichol is used at a concentration of between 0.001 and 7% by weight, preferably between 0.02 and 5% by weight, and may be associated with other fat-soluble vitaminic active ingredients with an anti-radical action such as Vitamin E and Vitamin F, which stabilise it and prolong its effects over time, and/or with plant polyprenoids from which dolichol can be synthesised.

The compositions of the invention are prepared according to techniques and with excipients known in dermatology and cosmetics, such as those described in Remington's Pharmaceutical Handbook, 17th ed., Mack Pub., N.Y., U.S.A., and may be presented in the form of creams, lotions, milks, fatty ointments, oils, ampoules, sticks and sprays, the sole limitation being the solubility characteristics of dolichol. The preferred propellant for the spray is carbon dioxide.

Dermatological compositions in paraffin and oils, which are well tolerated and do not cause irritation due to the active constituent or its interactions with the vehicle, even at the highest dolichol concentrations, are particularly preferred.

The invention will be illustrated below by means of the following examples.

### Examples

In examples 1-5, the amounts are expressed in grams.

### Example 1 - Cream

| | |
|---|---|
| Dehymuls F^{®} | 9 |
| Oleyl eructate | 6 |
| Decyl oleate | 6 |
| Isooctyl stearate | 5 |
| White vaseline | 10 |
| Dolichol | 0.2 |
| Glycerin | 3 |
| Magnesium sulphate | 0.3 |
| Water | 60.5 |
| | 100 |

### Example 2 - Cream

| | |
|---|---|
| Dehymuls K^{®} | 8 |
| Triglycerides of caprylic acid and lauric | 4 |
| acid | |
| Decyl oleate | 6 |
| Vegetable oil | 6 |
| White vaseline | 9 |
| Paraffin oil | 6 |
| Dolichol | 0.5 |
| Glycerin | 3 |
| Vitamin E | 0.5 |
| Vitamin F | 0.5 |
| Magnesium sulphate | 0.3 |
| Water | 56.2 |
| | 100 |

### Example 3 - Cream

| | |
|---|---|
| Glyceryl monostearate | 16 |
| Cetyl alcohol | 1 |
| Mineral oil | 10 |
| Sesame oil | 9 |
| Glycerin | 7 |
| Vitamin E | 1 |
| Dolichol | 1 |
| Water | 55 |
| | 100 |

### Example 4 - Liquid emulsion

| | |
|---|---|
| Cutin MD^{®} | 7 |
| Cetyl stearyl alcohol | 3 |
| Cetiol SN^{®} | 11 |
| Triglycerides of caprylic acid and capric | 11 |
| acid | |
| Dolichol | 0.1 |
| Water | 67.9 |
| | 100 |

### Example 5 - Spray solution

| | |
|---|---|
| Octyl dodecanol | 55 |
| Vegetable oil | 20 |
| Paraffin oil | 23 |
| Dolichol | 2 |
| | 100 |

The solution is mixed with carbon dioxide to give a 50:50 mixture.

### Example 6 - Study of anti UV-B activity

After irradiation, dolichol undergoes transformations in the most dangerous spectral region (UVB), depending on the dose of radiation applied; it absorbs UV radiation and, if irradiated with UVC rays, instantly acquires an absorption capacity in the UVB region. This phenomenon leads to a very rapid increase in absorbance at 320 nm (up 430% in one minute; up 580% in two minutes), with more modest increases in absorbance at 250 nm. The phenomenon is very weak in the case of irradiation with monochromatic light at 310 nm, more intense with irradiation at 280 nm, and greatest with white light irradiation, namely with the entire emission spectrum of the xenon lamp. It has been demonstrated that due to the effect of UV photo-irradiation in an oxygen atmosphere, the dolichol molecules undergo an apparent breakdown, accompanied by disappearance of UVC absorption capacity and therefore by consumption of the substance. The photodecomposition products of dolichol have not yet been identified but, by analogy with the findings made with peroxidative degradation of dolichol in isolated hepatocytes treated with carbon tetrachloride, it can be concluded that in biological systems, the degradation cascade leads to the formation of thiobarbituric acid reactive substances (TBARS), which are readily metabolised and detoxified by the body in the event of resorption. On the basis of theoretical knowledge, strengthened by the demonstration that the degradation of dolichol and the formation of TBARS in hepatocytes treated with CCl₄ is prevented by administering Vitamin E, the existence of some interesting positive interactions between the photoprotective effects of dolichol and those of Vitamin E can be postulated. The release by dolichol of biologically active substances, according to the extent of the photo-irradiation, suggests that it plays a complex role in controlling the natural adaptation mechanisms of the skin and its defences against the effects of sunlight. As regards absorption, it should be borne in mind that dolichol is a normal constituent of all cell membranes, in which it is synthesised and remains until breakdown, without being resorbed into the bloodstream or being transferable to other tissues.

*In vivo* photoprotection tests using dolichol (approx. 1 mg/cm2) demonstrate that the substance does not have cell-damaging effects or (photo)sensitising effects.

## Claims

1. Use of dolichol as an active agent for the preparation of sunscreen compositions for the prevention of acute and chronic skin damage caused by exposure to sunlight.

2. The use according to claim 1, wherein dolichol concentration is between 0.001 and 7% by weight.

3. The use according to claim 2, wherein dolichol concentration is between 0.02 and 5% by weight.

4. The use according to any one of the preceding claims, wherein dolichol is associated with other fat-soluble vitaminic active ingredients possessing an anti-radical action and/or with plant polyprenoids.

5. The use according to claim 4, wherein the fat-soluble vitaminic active ingredients with an anti-radical action are Vitamin E and Vitamin F and/or plant polyprenoids.

6. The use according to any one of claims 1-5, the composition being in the form of creams, lotions, milks, ointments, oils, ampoules, sticks and sprays.

7. The use according to claim 6, the composition being in spray form.

8. The use according to claim 7, wherein the propellant is carbon dioxide.

## Patentansprüche

1. Verwendung von Dolichol als einen aktiven Wirkstoff für die Herstellung von Sonnencremezusammensetzungen zum Schutz gegenüber plötzlich auftretenden und chronischen Schäden der Haut, welche durch Einwirkung von Sonnenlicht hervorgerufen werden.

2. Verwendung nach Anspruch 1, worin die Konzentration an Dolichol zwischen 0,001 und 7 Gew.-% liegt.

3. Verwendung nach Anspruch 2, worin die Konzentration an Dolichol zwischen 0,02 und 5 Gew.-% liegt.

4. Verwendung nach einem der vorherigen Ansprüche, worin Dolichol mit anderen fettlöslichen aktiven Vitamininhaltsstoffen, welche eine Wirkung gegenüber Radikale zeigen, und/oder mit Pflanzenpolyprenoiden kombiniert wird.

5. Verwendung nach Anspruch 4, worin die fettlöslichen aktiven Vitamininhaltsstoffe, welche eine Wirkung gegenüber Radikale zeigen, Vitamin E und Vitamin F und/oder Pflanzenpolyprenoide sind.

6. Verwendung nach einem der Anspruch 1 bis 5, worin die Zusammensetzung in der Form von Cremes, Lotionen, Milch, Salben, Ölen, Ampullen, Sticks und Sprays vorliegt.

7. Verwendung nach Anspruch 6, worin die Zusammensetzung in Sprayform vorliegt.

8. Verwendung nach Anspruch 7, worin das Treibgas Kohlendioxid ist.

## Revendications

1. Utilisation de dolichol en tant qu'agent actif pour la préparation de compositions de protection solaire pour la prévention d'un endommagement de la peau aigu et chronique provoqué par une exposition à de la lumière solaire.

2. Utilisation selon la revendication 1, dans laquelle la concentration de dolichol est entre 0,001 et 7 % en poids.

3. Utilisation selon la revendication 2, dans laquelle la concentration de dolichol est entre 0,02 et 5 % en poids.

4. Utilisation selon l'une quelconque des revendications précédentes, dans laquelle le dolichol est associé avec d'autres ingrédients actifs vitaminiques solubles dans une matière grasse, possédant une action antiradicalaire et/ou avec des polyprénoïdes de plante.

5. Utilisation selon la revendication 4, dans laquelle les ingrédients actifs vitaminiques solubles dans une matière grasse, ayant une action antiradicalaire, sont la vitamine E et la vitamine F et/ou des polyprénoïdes de plante.

6. Utilisation selon l'une quelconque des revendications 1 à 5, la composition étant sous la forme de crèmes, de lotions, de laits, de pommades, d'huiles, d'ampoules, de bâtons et de pulvérisations.

7. Utilisation selon la revendication 6, la composition étant sous forme de pulvérisation.

8. Utilisation selon la revendication 7, dans laquelle le propulseur est le dioxyde de carbone.
